# FASCICULE DE BREVET EUROPEEN

(11) **EP 3 174 549 B1**
(45) Date de publication et mention de la délivrance du brevet: **13.03.2019**
(21) Numéro de dépôt: 15767202.3
(22) Date de dépôt: 31.07.2015
(51) Int. Cl.: A61K 38/10, A61K 38/17, A61P 27/00, A61P 27/06, A61P 35/00

(54) **OLIGOPEPTIDES PARTICULIERS COMME MEDICAMENTS ANTI-ANGIOGENIQUES**
SPEZIFISCHE OLIGOPEPTIDE ALS ANTIANGIOGENE ARZNEIMITTEL
SPECIFIC OLIGOPEPTIDES AS ANTI-ANGIOGENIC DRUGS

(30) Priorité: 31.07.2014 FR 1457407
(43) Date de publication de la demande: 07.06.2017
(73) Titulaire: Neuronax, 63360 Saint-Beauzire (FR); Université de Limoges, 87032 Limoges (FR)
(72) Inventeur: GOBRON, Stéphane, F-63111 Dallet (FR); LALLOUE, Fabrice, F-87170 Isle (FR); BIBES, Romain, 1080 Uccle (BE); VINCENT, François, F-87220 Boisseuil (FR)
(74) Mandataire: Aquinov
(86) Numéro de dépôt international: PCT/FR2015/052133
(87) Numéro de publication internationale: WO 2016/016593

(56) Documents cités:
- WO-A1-2009/027350
- FR-A1- 2 766 190
- Lalloue F. (1), Domballe L. (1), Mitais N. (2), Bessette B. (1), Meiniel A. (2) & Jauberteau M. O. (1): "Dual role of TSR peptide on nervous and vascular system", , 2008, XP002734571, Extrait de l'Internet: URL:http://fens2008.neurosciences.asso.fr/ abstracts/Rpdf6/A184_35.pdf [extrait le 2015-01-13]
- SAKKA LAURENT ET AL: "SCO-spondin derived peptide NX210 induces neuroprotection in vitro and promotes fiber regrowth and functional recovery after spinal cord injury.", PLOS ONE 2014, vol. 9, no. 3, E93179, 25 mars 2014 (2014-03-25), pages 1-8, XP002734572, ISSN: 1932-6203
- GOBRON S ET AL: "Subcommissural Organ/Reissner's Fiber Complex: Characterization of SCO-Spondin, a Glycoprotein With Potent Activity on Neurite Outgrowth", GLIA, WILEY-LISS, NEW YORK, NY, US, vol. 32, 1 janvier 2000 (2000-01-01), pages 177-191, XP002442567, ISSN: 0894-1491, DOI: 10.1002/1098-1136(200011)32:2<177::AID-GLI A70>3.0.CO;2-V
- TUCKER RICHARD P: "The thrombospondin type 1 repeat superfamily.", THE INTERNATIONAL JOURNAL OF BIOCHEMISTRY & CELL BIOLOGY JUN 2004, vol. 36, no. 6, juin 2004 (2004-06), pages 969-974, XP002734573, ISSN: 1357-2725
- SORBERA L A ET AL: "ABT-510: Oncolytic angiogenesis inhibitor", DRUGS OF THE FUTURE, PROUS SCIENCE, ES, vol. 30, no. 11, 1 janvier 2005 (2005-01-01), pages 1081-1086, XP002460382, ISSN: 0377-8282, DOI: 10.1358/DOF.2005.030.11.949588
- SULOCHANA K N ET AL: "Developing antiangiogenic peptide drugs for angiogenesis-related diseases", CURRENT PHARMACEUTICAL DESIGN, BENTHAM SCIENCE PUBLISHERS, NL, vol. 13, no. 20, 1 janvier 2007 (2007-01-01), pages 2074-2086, XP009089402, ISSN: 1381-6128, DOI: 10.2174/138161207781039715

## Description

La présente invention concerne un oligopeptide particulier et son utilisation comme médicament dans le traitement de maladies associées à l'angiogenèse chez l'homme ou l'animal, en particulier comme agent anti- tumoral.

L'angiogenèse est le mécanisme responsable de la formation de nouveaux vaisseaux à partir de vaisseaux préexistants. Elle est indispensable au cours de certains processus physiologiques, en particulier pour la mise en place du système vasculaire chez l'embryon et pour l'implantation du placenta, mais elle peut également être responsable de développements pathologiques, comme la polyarthrite rhumatoïde, la dégénérescence maculaire chez l'adulte, et surtout la croissance des tumeurs et le développement des métastases. Il est en effet bien établi que le développement d'une vascularisation intra- ou péri-tumorale est un événement clé pour la croissance d'une tumeur et pour la dissémination métastatique par la voie sanguine.

C'est pourquoi, depuis plusieurs années, des recherches ont été menées sur des molécules présentant des effets anti-angiogéniques susceptibles d'être utilisées dans le traitement des cancers.

La plupart des agents anti-angiogéniques actuels ciblent principalement la cascade du VEGF (« Vascular Endothelial Growth Factor », facteurs de croissance endothélial vasculaire) produits par les cellules tumorales et sa liaison aux récepteurs notamment en utilisant des anticorps bloquant, ou des inhibiteurs des récepteurs à tyrosine kinases impliqués dans l'angiogenèse et la prolifération tumorale.

Toutefois, bien que le VEGF soit reconnu comme un facteur régulateur important dans la croissance tumorale et qu'il constitue une cible privilégiée pour les thérapies anticancéreuses, la mise au point de nouveaux agents inhibiteurs de l'angiogenèse représente un enjeu important pour accroître le nombre d'options thérapeutiques. De plus, l'identification de tels agents agissant sur des voies de la néo- ou l'anti-angiogenèse représenterait un progrès important pouvant permettre de développer un arsenal thérapeutique afin d'adresser de nombreuses pathologies dans lesquelles ces phénomènes d'angiogenèse interviennent (dégénérescence maculaire par exemple).

Si certains des inhibiteurs de l'angiogenèse développés actuellement montrent des effets sur le contrôle de la croissance des tumeurs en association avec une chimiothérapie conventionnelle, ils ne sont toutefois pas suffisamment efficaces, en particulier pour le traitement des tumeurs solides d'une façon générale et des tumeurs du système nerveux central plus spécifiquement. En effet, certaines de ces molécules, comme les anticorps, peuvent être des molécules présentant un poids moléculaire élevé qui les empêche de diffuser sur des distances importantes en particulier dans une masse tumorale ou de passer facilement la barrière hémato-encéphalique.

De plus, les molécules anti-angiogéniques existantes sont des protéines de synthèse qui sont complexes et couteuses à fabriquer. Elles doivent en outre pour la plupart être utilisées en forte concentration et présentent des effets secondaires importants comme par exemple l'Avastin® (bevacizumab). Ces effets secondaires, parfois d'issue fatale, rencontrés avec le bevacizumab sont notamment une leucopénie à l'origine d'infections ainsi qu'un risque hémorragique. D'autres approches de blocage de la voie du VEGF (anticorps anti-VEGF, anti-VEGF-R, petites molécules bloqueurs de PI3K entre autres) sont par ailleurs développées mais elles présentent dans la grande majorité des cas des limites en terme d'efficacité, de spécificité et d'effets indésirables, ces derniers pouvant porter sur le système cardiovasculaire. Des survenues d'hypertension artérielle sont ainsi généralement observées avec les différents anti-angiogéniques et des toxicités myocardiques pour certains d'entre eux *(*Wu S et al. Lancet Oncol 2008; 9: 407-11 *;* Nalluri SR, et al. JAMA 2008; 300(19): 2277-85*;* Chu TF, et al. Lancet 2007; 370: 2011-19*.).*

Il subsiste donc un besoin pour un agent anti-angiogénique palliant ces nombreux inconvénients.

Pour y répondre l'invention propose un oligopeptide particulier dont la séquence est déduite d'une partie des motifs TSR (« Thrombospondin type 1 repeat ») présents dans la SCO-spondine, glycoprotéine spécifique du système nerveux central et présente chez tous les vertébrés, des prochordés à l'homme. Il s'agit d'une molécule des matrices extracellulaires sécrétée par un organe particulier situé dans le toit du 3^{ème} ventricule, l'organe sous-commissural. La SCO-spondine est une molécule de grande taille, composée de plus de 4500 acides aminés et présentant une organisation multi-modulaire qui comprend différents motifs protéiques conservés dont en particulier 26 motifs TSR. On sait que certains peptides déduits de la SCO-spondine à partir des motifs TSR présentent une activité biologique sur les cellules nerveuses (notamment décrit dans la demande WO-99/03890) et que ces peptides agissent sur les cellules nerveuses par l'intermédiaire d'un récepteur spécifique (M. Bambad et al., Cell & Tissue Research, vol. 315, 15-25 (2004*)*).

Les motifs TSR sont des domaines protéiques d'environ 55 résidus, basés sur l'alignement d'acides aminés conservés cystéine, tryptophane et arginine. Ces motifs ont tout d'abord été isolés dans la TSP-1 (thrombospondine 1) qui est une molécule intervenant dans la coagulation. Ils ont ensuite été décrits dans de nombreuses autres molécules aux fonctions biologiques diverses telles que l'attachement cellulaire, la mobilité, la prolifération, l'agrégation cellulaire, la modulation de protéases ou l'inhibition de l'angiogenèse.

La TSP-1 est une glycoprotéine de la matrice extracellulaire d'un poids moléculaire d'environ 120 kDa dans sa forme monomérique, possédant un domaine de liaison à l'héparine et des motifs répétés de trois types qui peuvent se lier à un certain nombre de facteurs angiogènes, dont le FGF-2, le VEGF, le PDGF et le TGF-β1 *(Lamszus et al*., *1996; Margosio et al., 2003).* Cette interaction permet la séquestration de ces facteurs et limite leurs interactions avec leurs récepteurs respectifs. Le second mécanisme d'action de la TSP-1 passe notamment par sa liaison aux cellules endothéliales via les protéoglycanes, les intégrines, le CD47 et le CD36 *(Asch et al*., *1987; Gao et al*., *1996; Primo et al*., *2005*; *Zhang et al., 2009b).* L'interaction de TSP-1 avec CD36 pourrait inhiber la signalisation pro-angiogénique en formant un complexe avec NRP-1 et VEGFR-2 qui empêcherait l'activation du VEGFR-2 induite par le VEGF. La TSP-1 peut de plus agir sur la NOS, inhiber la migration des cellules endothéliales induites par le FGF-2 et également induire l'apoptose des cellules endothéliales. Par ailleurs, il a été décrit que les propriétés anti-angiogéniques de TSP1 impliquaient une inhibition de la survie, de la prolifération et de la migration des cellules endothéliales (Jiménez B, Volpert OV, Crawford SE, Febbraio M, Silverstein RL, Bouck N. Nat Med. 2000 Jan;6(1):41-8).

On sait d'une manière générale que certains fragments peptidiques de la TSP-1 ainsi que certains peptides synthétiques dont la structure est analogue à celle de ces fragments peptidiques montrent une activité similaire à la thrombospondine-1 native.

A titre d'exemple, les brevets US 6,239,110, US 5,840,692, US 5,849,701, US 5,491,130 et US 6,051,549, US 5,190,918 et US 5,200,397 et US 6,384,189 décrivent la structure et les effets biologiques de plusieurs de ces fragments peptidiques déduits de la TSP-1 et de ses analogues synthétiques.

En outre, un certain nombre de molécules à visée anti-angiogénique inspirées des motifs TSR de la TSP-1 ont été développées. On connaît plusieurs dérivés peptidiques ou peptides de ce type, présentés comme capables de mimer les effets de TSP-1, notamment l'ABT-510 et l'ABT-526. Toutefois, malgré des séquences en acides aminés et une affinité pour leur récepteur proches, la solubilité et l'effet de ces peptides de synthèse sur les cellules endothéliales peut varier (*Haviv et al*., *2005*). L'ABT-510 en particulier s'est révélé inducteur *in vitro* de l'apoptose dépendant de la Capsase-8 via CD36 et a réduit la vascularisation tumorale dans un modèle préclinique de greffe hétérotopique de gliome chez la souris nude (*Anderson et al. 2007*)*.* Il a par ailleurs fait l'objet d'études cliniques dans différents cancers, notamment le sarcome et le carcinome rénal. Toutefois, bien que son innocuité ait été démontrée, l'efficacité en monothérapie du composé ABT-510 a été jugée insuffisante (*Baker et al., 2008; Ebbinghaus et al*., *2007*)*.*

D'autres composés peptidiques ont été élaborés à partir de motifs TSR de la TSP-1 comme par exemple le 3TSR (*Ren et al*., *2009*), ou ont été étudiés en provenance d'autres protéines ayant des motifs TSR, comme par exemple ADAMTS5 à partir de laquelle une protéine recombinante portant des motifs TSR a été obtenue (*Sharghi-Namini et al*, *2008*)*.* Le mécanisme d'action de cette protéine recombinante reste toutefois VEGF-dépendant. En effet, la protéine recombinante a montré qu'elle inhibait la stimulation des cellules endothéliales par VEGF et induisait leur apoptose en présence de VEGF. Par ailleurs, l'obtention de cette protéine recombinante en quantité suffisante nécessite de faire appel à des systèmes d'expression qui peuvent être complexes pour une qualité pharmaceutique car pouvant introduire des contaminations ou modifier la conformation (structure tertiaire) de la protéine à produire.

Actuellement, les séquences des motifs TSR connus pour leur efficacité sont :
- la séquence V-T-C-G (SEQ ID n°2) qui peut se lier au récepteur CD36 ou gpIV des plaquettes ;
- la séquence C-S-V-T-C-G (SEQ ID n°3) : S.S. Tolsma et al. (The Journal of Cell Biology, vol. 122 p.497-511 (1993*)*) ont montré que l'activité anti-angiogénique de la TSP-1 pouvait être reproduite *in vitro* et *in vivo* avec différents peptides synthétiques, dont en particulier deux séquences déduites du domaine TSR et qui présentent en position centrale la séquence C-S-V-T-C-G (SEQ ID n°3). De plus des études ultérieures ont précisé que l'activité de néovascularisation par la TSP-1 pouvait se dérouler selon deux mécanismes différents qui faisaient intervenir deux régions différentes du TSR dont une région renfermant la séquence C-S-V-T-C-G (SEQ ID n°3) (M.L. Iruela-Arispe, Circulation, vol. 100 p1423-31 (1999*)*) ;
- la séquence W-S-X-W (SEQ ID n°4) qui est capable de lier l'héparine et des protéoglycanes permettant ainsi un effet biologique variable et intervenant dans le processus d'activation du TGFβ par la TSP-1. La présence de ce motif W-S-X-W (SEQ ID n°4) bien que non suffisante à elle seule, est nécessaire pour cette activation de la cytokine qui peut alors intervenir dans différents évènements biologiques tels que l'inflammation, l'angiogenèse ou le développement embryonnaire (G. D. Young et J. E. Murphy-Ullrich, The Journal of Biological Chemistry, vol. 279, p. 47633-47642 (2004*)*). De plus, ce motif semble agir comme compétiteur de certains facteurs de croissance pro-angiogéniques lors de leur liaison aux protéoglycanes présents à la surface de cellules endothéliales (T. Vogel et al., the Journal of Cellular Biochemistry, vol. 53 p.74-84 (1993*)*). Il s'agit également de la seconde région du TSR citée dans M.L. Iruela-Arispe (Circulation, vol. 100 p1423-31 (1999*)*) intervenant dans les mécanismes responsables de l'activité de néovascularisation par la TSP-1. Toutefois la constitution exacte de l'acide aminé variant (X) ou des acides aminés additionnels situés en amont ou en aval de ce motif est importante pour l'activité du peptide. C'est le cas en particulier de l'efficacité de la liaison à l'héparine et de l'induction de l'adhésion cellulaire qui se sont trouvées augmentées pour un pentapeptide où X étant la Proline ou des acides aminés basiques *((*N.H. Guo, The Journal of Biological Chemistry, vol. 267 p.19349-55 (1992*)*).

A ce jour, aucun produit anti-angiogénique suffisamment efficace et bien toléré n'a été développé et aucun produit construit sur la base de tous les éléments précédemment décrits n'a été testé pour des activités anti-angiogéniques.

C'est pourquoi l'invention propose un oligopeptide spécifique efficace et bien toléré, qui supprime certains inconvénients des thérapies anti-angiogéniques existantes.

En particulier, l'invention vise un oligopeptide présentant la séquence suivante :
-W-S-X₁₋W-S- X₂-C-S- X₃-X₄-C-G- (SEQ ID N°1)
dans lequel X₁, X₂, X₃ et X₄ représentent des séquences d'acides aminés consistant en 1 à 5 acides aminés, comme médicament inhibiteur de l'angiogenèse. Il est utile dans le traitement de maladies associées à l'angiogenèse choisie parmi les cancers, l'arthrite, la polyarthrite rhumatoïde, des plaques d'athérosclérose, la néovascularisation d'une greffe de cornée, des cicatrices hypertrophiques ou chéloïdes, la rétinopathie proliférante, la rétinopathie diabétique, la dégénérescence maculaire chez l'adulte, la granulation, le glaucome néovasculaire et l'uvéite.

Cette séquence d'acides aminés a été décrite sous sa forme -W-S-G-W-S-S-C-S-R-S-C-G-(SEQ ID N°5) dans le brevet WO9903890 et WO2009027350 mais aucunement pour des activités anti-angiogéniques.

De façon surprenante et inattendue, l'oligopeptide selon l'invention joue un rôle protecteur de l'apoptose des HUVECs ou des HBMECs, et ceci de façon comparable à une dose élevée de VEGF (50 ng/mL), et ne module pas leur prolifération. *ln vitro*, les effets de l'oligopeptide selon l'invention sur les cellules endothéliales semblent donc opposés à ceux de la TSP-1. Par ailleurs, l'oligopeptide selon l'invention favorise la migration en présence d'un facteur angiogènique tel que le VEGF ou le bFGF. Il inhibe cependant la migration des HBMECs induite par des cellules tumorales de glioblastome lorsqu'on le met au contact des U87-MG ou des HBMECs.

De plus, il a été démontré que contrairement à TSP-1, l'action de l'oligopeptide objet de la présente invention n'implique pas le récepteur CD36.

Il présente ainsi un rôle inhibiteur sur les mécanismes de vascularisation et un effet anti-angiogénique important qui corrèle l'effet anti-tumoral également observé. Du fait de sa petite taille, il est aussi particulièrement adapté au traitement des tumeurs du système nerveux central, notamment des glioblastomes.

L'oligopetide selon l'invention pour son utilisation comme anti-angiogénique est unique par ses effets et ses mécanismes d'action originaux. En effet, ABT-510 qui est la molécule anti-angiogénique la plus proche de par son origine, dérive de la partie centrale de la seconde répétition thrombospondine de type 1 située à l'extrémité N-terminale de la TSP-1, protéine exprimée par de nombreux types cellulaires et impliquée dans différents processus biologiques y compris l'angiogenèse, alors que l'oligopeptide selon l'invention représente la séquence consensus de la partie N-terminale des 27 répétitions TSR de la SCO-spondine qui est une protéine présentant des motifs TSR mais spécifiquement exprimée au niveau du SNC et intervenant notamment pendant le développement.

L'oligopeptide présente en outre une innocuité améliorée, l'oligopeptide selon l'invention ne montrant aucune toxicité même à des injections de 20mg/kg.

D'autres caractéristiques et avantages ressortiront de la description qui va suivre de l'invention, en regard des dessins annexés sur lesquels :
- les figures 1A à 1D représentent des observations macroscopiques (grossissement x20) réalisées après injection de fluorescéine aqueuse dans la circulation systémique d'un embryon à ED9 :
   ∘ figure 1A : eau (contrôle)
   ∘ figure 1B : bevacizumab (témoin négatif)
   ∘ figure 1C : VEGF à 10ng/mL (témoin positif)
   ∘ figure 1D : oligopeptide selon l'invention 250µg/mL
- les figures 2A à 2C représentent des résultats d'analyse de facteurs solubles intervenant dans l'angiogenèse :
   ∘ des surnageants de cellules U87-MG ont été étudiés par protéome array (figures 2A et 2B) pour la recherche de protéines impliquées dans l'angiogenèse, en présence d'eau stérile, de TSP-1 et de l'oligopeptide selon l'invention
   ∘ les sécrétions de VEGFA par les U87-MG dans le milieu après traitements avec l'eau stérile, TSP-1 et l'oligopeptide selon l'invention, ont été dosés par ELISA à partir des surnageants (figure 2C).

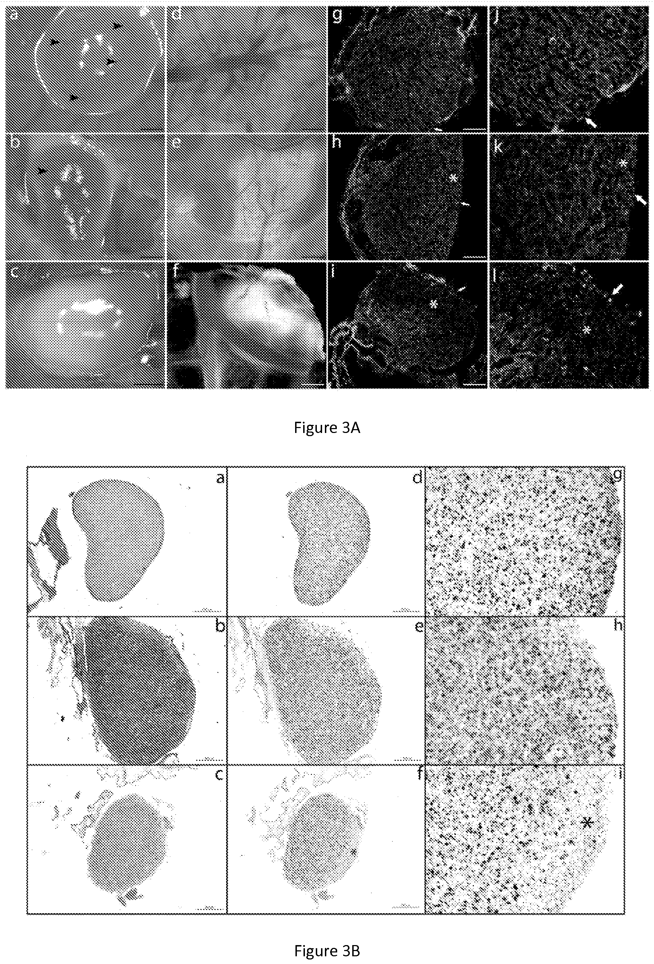

- les figures 3A à 3C représentent des résultats de l'analyse de l'effet de l'oligopeptide selon l'invention sur l'angiogenèse et la prolifération tumorale dans le modèle de la CAM :
   ∘ à partir de tumeurs issues de greffes de U87-MG sur la CAM, la vascularisation des tumeurs expérimentales a été étudiées aux niveaux macroscopiques (figure 3A - a à f) et microscopique (figure 3A g à l) après traitement pendant 48 heures avec :
      ▪ H₂O: a, d, g et j
      ▪ la TSP-1 : b, e, h et k
      ▪ l'oligopeptide selon l'invention : c, f, i et l

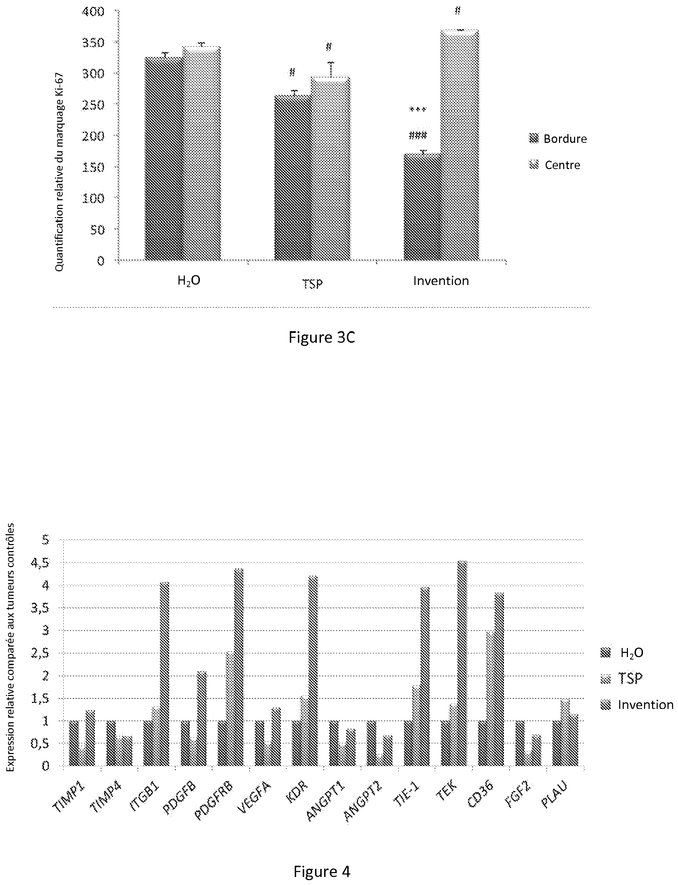

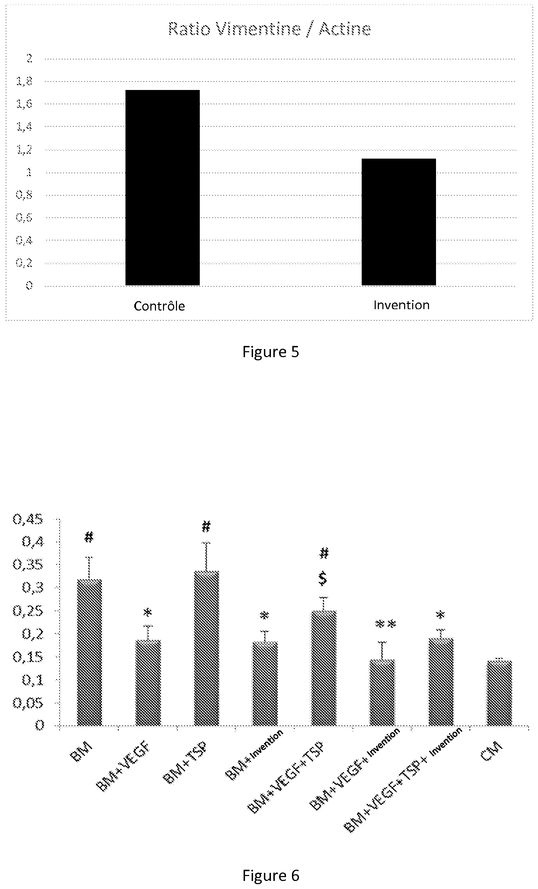

L'observation microscopique des vaisseaux est réalisée après marquage de ceux-ci par la SNA-1. Les barres d'échelle pour les images h et i de la figure 3A représentent 1 mm et les images j, k et l de la figure 3A sont des grossissements (x2) des images g, h et i de la figure 3A, respectivement ; les têtes de flèches montrent les vaisseaux visibles, les flèches montrent les surfaces traitées et les étoiles les zones sans vaisseaux.
∘ la structure des tumeurs a été visualisée après un marquage par hématoxyline, éosine et safran de coupes de tumeurs expérimentales (figure 3B a à c) et la prolifération des cellules tumorales a été étudiée par marquage immunohistochimique du Ki-67 avec contre-coloration par hématoxyline (figure 3B d à i). Les tumeurs ont été traitées 48 heures avec
   ▪ eau : a, d et g
   ▪ la TSP-1 : b, e et h
   ▪ l'oligopeptide selon l'invention : c, f et i
   les images g, h et i de la figure 3B sont des grossissements (x4) des bordures des images d, e et f de la figure 3B, respectivement. Les flèches montrent les surfaces traitées et les étoiles les zones sans marquage du Ki-67.
∘ Le marquage du Ki-67 a été quantifié (Figure 3C) dans les différentes conditions à l'aide du logiciel ImageJ et des comparaisons entre le centre et la bordure des tumeurs et entre les conditions ont été effectuées par analyse de variances (ANOVA). Une valeur de p<0,05 est considérée significative. * p<0,05; ** p<0.01 ; *** p<0.001 en comparaison au centre de la même tumeur. $ p<0,05 ; $$ p<0,01 ; $$$ p<0,001 en comparaison à la tumeur contrôle.
   - la figure 4 représente les résultats de l'analyse quantitative des ARNm dans des tumeurs expérimentales issues de la CAM, les différents gènes étant représentés par leur expression relative comparée aux tumeurs contrôles
   - la figure 5 représente les résultats de l'analyse semi-quantitative de l'expression protéique de la vimentine par western blot avec ou sans l'oligopeptide selon l'invention sur les cellules U87-MG
   - la figure 6 représente les résultats de l'analyse de l'effet fonctionnel de l'oligopeptide selon l'invention sur l'apoptose des HBMECs *in vitro.* Il s'agit d'une synthèse d'une analyse par ELISA Cell Death : les traitements par VEGF, TSP-1 et l'oligopeptide selon l'invention (NX) ont été effectués séparément ou on combinaison dans du milieu basal (BM).

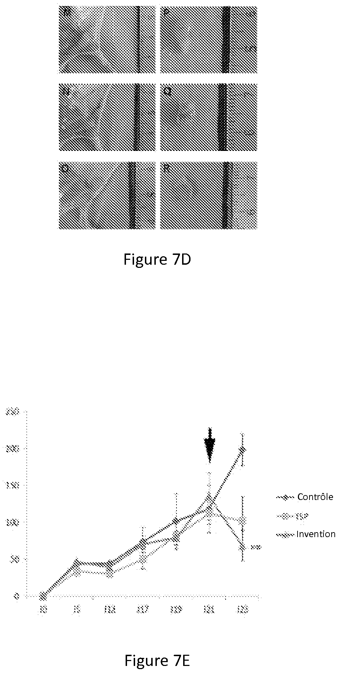

Du milieu complet (CM) et du milieu basal (BM) ont été utilisés comme contrôles. Une valeur de p<0,05 est considérée significative. Les comparaisons entre différents traitements ont été effectuées par analyse de variances (test ANOVA). * p<0.05; ** p<0.01 comparé à BM; $ p<0,05 comparé à BM+NX; # p<0,05 comparé à CM,

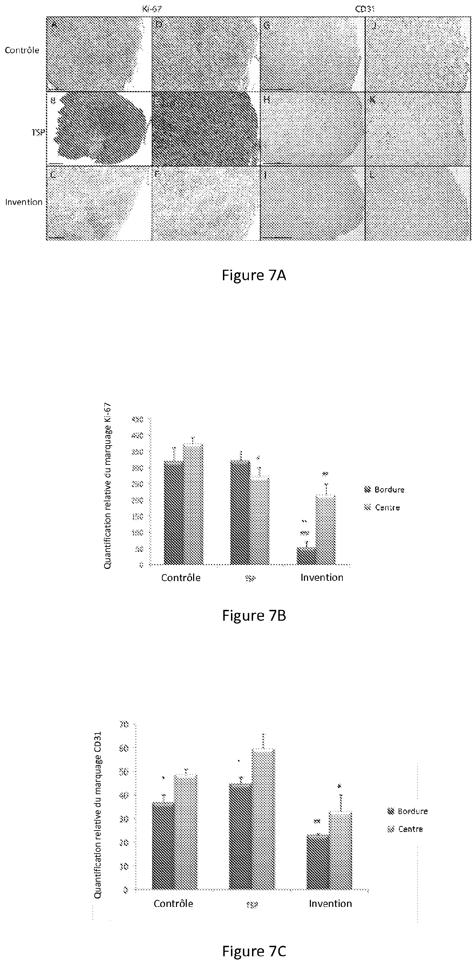

- les figures 7A à 7E représentent les résultats de l'analyse de l'activité de l'oligopeptide selon l'invention sur la prolifération, la vascularisation et la croissance tumorale chez la souris Nude :
   ∘ les images A, B et C de la figure 7A représentent l'immunomarquage du Ki-67 sur coupes de tumeurs traitées par H₂O, TSP-1 ou l'oligopeptide selon l'invention respectivement
   ∘ les images D, E et F de la figure 7A sont des grossissements (x2) des figures A, B et C
   ∘ les images G, H et I de la figure 7A représentent l'immunomarquage du CD31 sur coupes de tumeurs traitées par H₂O, TSP-1 ou l'oligopeptide selon l'invention respectivement (les barres d'échelle correspondent à 500µm)
   ∘ les images J, K et L de la figure 7A sont des grossissements (x2) des images G, H et I
   ∘ le marquage du Ki-67 a été quantifié dans les différentes conditions à l'aide du logiciel imageJ et des comparaisons entre le centre et la bordure des tumeurs et entre les conditions ont été effectuées (figure 7B) par analyse de variances (test ANOVA). Une valeur de p<0,05 est considérée significative. * p<0,05 comparé au centre des mêmes tumeurs ; $ p<0,05 et $$ p<0,01 comparé au contrôle
   ∘ le marquage du CD31 a été quantifié dans les différentes conditions à l'aide du logiciel imageJ et des comparaisons entre le centre et la bordure des tumeurs et entre les conditions ont été effectuées (figure 7C) par analyse de variances (test ANOVA). Une valeur de p<0,05 est considérée significative. * p<0,05 comparé au centre des mêmes tumeurs ; $ p<0,05 et $$ p<0,01 comparé au contrôle
   ∘ les images M et P de la figure 7D représentent des tumeurs de souris après traitement par hydrogel seul
   ∘ les images N et Q de la figure 7D représentent des tumeurs de souris après traitement par TSP-1 dans de l'hydrogel
   ∘ les images O et R de la figure 7D représentent des tumeurs de souris après traitement par l'oligopeptide selon l'invention (NX) dans de l'hydrogel
   ∘ la figure 7E représente les mesures des croissances tumorales traitées avec H₂O, TSP-1 et l'oligopeptide selon l'invention à 5, 12, 17, 19 21 et 23 jours après l'injection de cellules cancéreuses. Les injections des traitements (hydrogel seul, TSP-1 et l'oligopeptide selon l'invention (NX) ont été réalisées au 21^{e} jour (flèche noire). La comparaison des tailles tumorales en comparaison aux contrôles a été effectuée par analyse de variances (test ANOVA). ** p<0.01; *** p<0.001.

L'invention vise donc un oligopeptide présentant la séquence suivante :
-W-S-X₁-W-S- X₂-C-S- X₃-X₄-C-G- (SEQ ID N°1)
dans lequel X₁, X₂, X₃ et X₄ représentent des séquences d'acides aminés consistant en de 1 à 5 acides aminés, comme médicament inhibiteur de l'angiogenèse. Il est utile dans le traitement d'au moins une maladie associée à l'angiogenèse, c'est-à-dire une maladie caractérisée par une angiogenèse excessive ou anormale ou par une prolifération des cellules endothéliales, choisie parmi : les cancers, l'arthrite, la polyarthrite rhumatoïde, des plaques d'athérosclérose, la néovascularisation d'une greffe de cornée, des cicatrices hypertrophiques ou chéloïdes, la rétinopathie proliférante, la rétinopathie diabétique, la dégénérescence maculaire chez l'adulte, la granulation, le glaucome néovasculaire et l'uvéite.

Au sens de la présente invention, on entend par acides aminés les acides aminés naturels et les acides aminés non naturels.

Par « acides aminés naturels » on entend la forme L des acides aminés qui peuvent être trouvés dans des protéines d'origine naturelle, c'est-à-dire : alanine (A), arginine (R), asparagine (N), acide aspartique (D), cystéine (C), glutamine (Q), acide glutamique (E), glycine (G), histidine (H), isoleucine (I), leucine (L), lysine (K), méthionine (M), phénylalanine (F), proline (P), sérine (S), thréonine (T), tryptophane (W), tyrosine (Y) et valine (V).

Par « acides aminés non naturels » on entend la forme D des acides aminés naturels, les formes *homo* de certains acides aminés naturels (tels que : arginine, lysine, phénylalanine et sérine), et les formes nor de la leucine et de la valine. Ils comprennent aussi des acides aminés synthétiques comme l'acide alpha-aminobutyrique (Abu), l'agmatine (Agm), l'acide alpha-arinoisobutyrique (Aib), la N-formyle-trp (F-trp), la sarcosine , la statine, l'ornithine, la désaminotyrosine. La désaminotyrosine est incorporée à l'extrémité N-terminale alors que l'agmatine et la statine sont incorporées à l'extrémité C-terminale de ces peptides.

Dans la séquence SEQ ID n°1, au moins un acide aminé peut être remplacé par un de ses dérivés ou analogues.

Selon un mode de réalisation préféré, X₁, X₂, X₃ et X₄ représentent chacun un acide aminé.

Préférentiellement X₁ est choisi parmi G, V, S, P et A, X₂ est choisi parmi G, V, S, P et A, X₃ est choisi parmi R, A et V et X₄ est choisi parmi S et P. La séquence correspondante est la séquence SEQ ID n°6 : W-S-X₁-W-S- X₂-C-S- X₃-X₄-C-G- dans laquelle est choisi parmi G, V, S, P et A, X₂ est choisi parmi G, V, S, P et A, X₃ est choisi parmi R, A et V et X₄ est choisi parmi S et P.

Selon une variante X1 et X2 peuvent être W-S-S-, W-S-G-, W-S-P-, W-S-A-, W-S-R-. La séquence correspondante est la séquence SEQ ID n°7 : -W-S-X₁-W-S- X₂-C-S- X₃-X₄-C-G- dans laquelle X1 et X2 sont choisis parmi W-S-S-, W-S-G-, W-S-P-, W-S-A-, W-S-R- et X₃ et X₄ représentent des séquences d'acides aminés consistant en de 1 à 5 acides aminés. L'oligopeptide peut être modifié par amidation, acylation, pegylation, par addition d'acides aminés, mais également par modification en staple peptides ou par tout procédé de conjugaison notamment avec l'albumine ou des polymères comme le PEG, ou combinaison avec un gel, hydrogel. L'oligopeptide peut également être cyclisé.

L'oligopeptide selon l'invention peut être obtenu de différentes façons : par synthèse chimique en phase liquide ou sur support solide (Fmoc par exemple), par d'autres méthodes faisant appel aux techniques recombinantes et de constructions génétiques comportant une séquence ou un fragment d'ADN pouvant coder pour des composés peptidiques comportant la séquence de l'oligopeptide selon l'invention et permettant de recueillir par des systèmes d'expression eucaryotes ou procaryotes le produit d'expression ou d'exprimer *in situ* le produit. L'oligopeptide selon l'invention peut se présenter sous forme déglycosylée ou bien glycosylée si cela est nécessaire. Dans certains cas, et suivant la méthode de préparation, il pourra être nécessaire de renaturer certaines structures tertiaires de l'oligopeptide.

L'oligopeptide selon l'invention :
- joue un rôle protecteur de l'apoptose des HUVECs ou des HBMECs et ne module pas leur prolifération
- favorise la migration en présence d'un facteur angiogènique tel que le VEGF ou le bFGF
- inhibe la migration des HBMECs induite par des cellules tumorales de glioblastome lorsqu'on le met au contact des U87-MG.

L'oligopeptide selon l'invention peut donc être utilisé comme principe actif thérapeutique ou médicament anti-angiogénique dans le traitement de différentes pathologies notamment les cancers, l'arthrite, la polyarthrite rhumatoïde, des plaques d'athérosclérose, la néovascularisation d'une greffe de cornée, des cicatrices hypertrophiques ou chéloïdes, la rétinopathie proliférante, la rétinopathie diabétique, la dégénérescence maculaire chez l'adulte, la granulation, le glaucome néovasculaire et l'uvéite.

Avantageusement, il est capable d'inhiber la néo-angiogenèse de tumeurs chez l'homme ou l'animal.

Selon un autre avantage, la petite taille de l'oligopeptide de séquence SEQ ID N°1 facilite le passage de la barrière hémato-encéphalique et la bio-distribution rapide dans le système nerveux central ainsi que le ciblage de la zone tumorale. Sa solubilité en solutions aqueuses le rend compatible avec la pratique clinique. Il peut donc être utilisé comme médicament pour le traitement des tumeurs cérébrales chez l'homme ou l'animal, en particulier pour le traitement de glioblastomes.

Par ailleurs, comme il s'agit d'un oligopeptide issu d'une protéine présente dans l'organisme, il présente une tolérance accrue et des propriétés d'induction d'effets secondaires indésirables restreintes par rapport à des molécules résultats de synthèse chimique ou issues de plantes comme c'est le cas de nombreux anti-angiogéniques actuels. De plus, il est soluble, non toxique, et lorsqu'il est constitué d'acides aminés naturels, il est non immunogène et est facilement éliminé par l'organisme.

Le mode d'action de l'oligopeptide selon l'invention est indépendant du CD36, contrairement à celui de la TSP-1. En effet, lorsque le CD36 est activé par la TSP-1 dans les cellules endothéliales, il interfère avec l'activation du VEGF-R2 (KDR) et induit l'apoptose des cellules endothéliales. L'oligopeptide selon l'invention favorise le rapprochement entre les deux récepteurs (CD36 et VEGFR-2), mais augmente l'expression de CD36. Plutôt que de jouer un rôle inhibiteur directement sur les cellules endothéliales, l'oligopeptide selon l'invention favorise l'activation du VEGFR-2 en présence de son ligand, la voie du VEGF étant connue comme favorisant la survie des cellules endothéliales et leur migration.

Par ailleurs l'oligopeptide selon l'invention est capable de moduler deux molécules : CD36 et VEGF-R2 participant au phénomène d'angiogenèse.

L'oligopeptide selon l'invention peut être administré par tout mode d'administration adapté. Pour le traitement de maladies touchant le système nerveux central, il est préférentiellement administré par voie intrathécale, intraventriculaire, intraparenchymateuse, épidurale. Pour les autres applications, il est préférentiellement administré par voie intraveineuse, par voie intraluminale, par voie intravitréale, par voie transphénoïdale ou par voie topique, ces modes d'administration n'étant pas limitatifs.

Il peut être intégré dans des compositions pharmaceutiques sous une forme quelconque, notamment liquide ou gel ou tout autre support, en particulier permettant libération contrôlée.

L'oligopeptide est préférentiellement formulé dans une composition pharmaceutique, en particulier une composition pharmaceutique se présentant sous forme
- liquide : il peut notamment être formulé dans des préparations injectables sous forme de solutions, suspensions ou émulsions (préparations pour perfusion),
- ou solide, et comporter tout type de support, en particulier gels, biopolymères ou biomatériaux ou tout dispositif médical comme les implants ou les pompes implantables par exemple, permettant une libération contrôlée ou bien un système de vectorisation.

Dans le cadre du traitement de tumeurs, l'oligopeptide selon l'invention peut éventuellement être utilisé seul ou en association avec un ou plusieurs autres principes actifs anti-tumoraux. Il peut être utilisé avec au moins un principe actif chimiothérapeutique anti-cancéreux par exemple.

L'invention vise donc également les compositions pharmaceutiques comprenant un oligopeptide présentant la séquence suivante :
-W-S-X₁-W-S- X₂-C-S- X₃-X₄-C-G- (SEQ ID N°1)
dans lequel X₁, X₂, X₃ et X₄ représentent des séquences d'acides aminés consistant en 1 à 5 acides aminés, tel que décrit précédemment pour son utilisation comme médicament inhibiteur de l'angiogenèse, ces compositions étant utiles dans le traitement de maladies associées à l'angiogenèse **choisie parmi les cancers, l'arthrite, la polyarthrite rhumatoïde, des plaques d'athérosclérose, la néovascularisation d'une greffe de cornée, des cicatrices hypertrophiques ou chéloïdes, la rétinopathie proliférante, la rétinopathie diabétique, la dégénérescence maculaire chez l'adulte, la granulation, le glaucome néovasculaire et l'uvéite.**

L'invention est à présent illustrée par un exemple d'oligopeptide selon l'invention et par des résultats d'essais démontrant l'effet anti-angiogénique de l'oligopeptide selon l'invention, en particulier de l'oligopeptide de l'exemple 1.

### EXEMPLE :

-W-S-G-W-S-S-C-S-R-S-C-G- (SEQ ID N°5)
Cet oligopeptide objet de la présente invention correspond aux 12 acides aminés les plus conservés issus du quatrième domaine TSR de la SCO-spondine.

### ESSAIS

### A. Etude des effets de l'oligopeptide selon l'invention sur l'angiogenèse in vitro

Le processus d'apoptose s'accompagne de la fragmentation de l'ADN et génère ainsi des nucléosomes solubles (histones cytoplasmique associées à l'ADN fragmenté) qui peuvent être dosés par une méthode immunoenzymatique, ELISA Cell Death Plus (Enzyme-Linked ImmunoSorbent Assay) Kit (Roche Molecular Diagnostics, France) selon les recommandations du fournisseur. Les cellules HBMECs ont été ensemencées en plaque 24 puits à la densité de 5.10³ cellules / puits et laissées 24 heures au repos. Les cellules sont cultivées dans un milieu basal c'est-à-dire en absence de facteurs de croissance qui est changé avant qu'elles soient exposées aux différents traitements pendant 24 heures. Elles sont ensuite récupérées puis centrifugées à 200g pendant 5 min. Les cellules sont alors comptées, puis ajustées à 3.10⁴ cellules/mL dans du PBS. Une nouvelle centrifugation à 200 g est réalisée pendant 10 min. Le surnageant est retiré et le culot cellulaire est repris dans 200 µL de tampon de lyse avant une incubation de 30 min à température ambiante. Après une nouvelle centrifugation de 10 min à 1400 rpm, 20 µL de surnageant sont distribués dans les puits coatés à la streptavidine et chaque condition est réalisée en duplicat. 80 µL de la solution d'immunoréactif sont ajoutés dans chaque puits. Cette solution contient du tampon d'incubation ainsi que 2 anticorps : un anticorps anti-histone couplé à la biotine et un anticorps anti-ADN couplé à la HRP (Horse Radish Peroxydase). Les 2 anticorps vont se lier à la plaque via la streptavidine. La plaque est incubée pendant 2h à température ambiante, sous agitation et à l'obscurité. Après cette étape, 3 lavages avec 200 µL de tampon d'incubation sont réalisés afin d'éliminer l'excès d'anticorps non fixés. 100 µL d'ABTS, qui est le substrat de la HRP, sont ensuite rajoutés dans chaque puits. L'incubation avec la solution d'ABTS est effectuée à l'obscurité pour une durée de 15 à 20 min. La réduction de l'ABTS par l'HRP permet la visualisation de l'apoptose par réaction colorimétrique. La réaction est alors stoppée par ajout de 100 µL de solution stop ABTS puis les résultats sont analysés grâce à un lecteur de plaque à 405 nm. La quantité d'oligonucléosomes formée est proportionnelle à l'intensité de la coloration et à l'apoptose cellulaire.

Les résultats obtenus, présentés sur la figure 6 et dans le tableau 1 ci-après :

**Tableau 1 : Valeurs moyennes relatives aux densités optiques mesurées pour chaque condition de traitement lors du test d'induction de l'apoptose sur les cellules HBMEC**

| Conditions | Valeurs Densité Optique | Ecart moyen |
|---|---|---|
| BM | 0,259 | |
| BM | 0,414 | |
| BM | 0,279 | 0,06444444 |
| BM+VEGF | 0,234 | |
| BM+VEGF | 0,133 | |
| BM+VEGF | 0,195 | 0,03622222 |
| BM+TSP | 0,215 | |
| BM+TSP | 0,419 | |
| BM+TSP | 0,372 | 0,08022222 |
| BM+NX | 0,199 | |
| BM+NX | 0,138 | |
| BM+NX | 0,21 | 0,02955556 |
| BM+VEGF+TSP | 0,214 | |
| BM+VEGF+TSP | 0,235 | |
| BM+VEGF+TSP | 0,304 | 0,03533333 |
| BM+VEGF+NX | 0,217 | |
| BM+VEGF+NX | 0,118 | |
| BM+VEGF+NX | 0,097 | 0,04866667 |
| BM+VEGF+TSP+NX | 0,212 | |
| BM+VEGF+TSP+NX | 0,206 | |
| BM+VEGF+TSP+NX | 0,154 | 0,02444444 |
| CM | 0,14 | |
| CM | 0,135 | |
| CM | 0,15 | 0,00555556 |

| | | |
|---|---|---|
| *BM = milieu basal* *CM = milieu complet* *NX = oligopeptide selon l'invention* | | |

Ces résultats montrent que l'oligopeptide selon l'invention protège le cellules endothéliales humaines HBMECs de l'apoptose à un niveau similaire au VEGF, et cet effet n'est pas observé en présence de TSP-1. En fait, TSP-1 est connu pour induire l'apoptose des cellules endothéliales. Par ailleurs, le traitement combiné oligopeptide selon l'invention + VEGF améliore cet effet protecteur.

### B. Etude des effets du peptide selon l'invention sur l'angiogenèse à partir d'un modèle aviaire de membrane chorio-allantoïdienne (CAM)

L'angiogenèse est l'un des mécanismes clé du développement embryonnaire. C'est également un processus déterminant sous certaines conditions physiologiques et pathologiques (cancers en particulier). Ce mécanisme est sous la dépendance de nombreux facteurs possédant soit un potentiel inhibiteur, soit activateur. Afin d'évaluer les principales propriétés de ces facteurs *in vivo*, le modèle le plus répandu est le modèle de la CAM (membrane chorio-allantoïdienne) à partir de l'embryon de poulet (Ribatti et *al*., 2000). Il présente l'avantage de permettre de tester les facteurs pro ou anti-angiogéniques lors d'une phase d'angiogenèse active entre le stade embryonnaire 4j et 10j. Les effets majeurs des différentes molécules peuvent donc être évalués *in vivo.* Le modèle de la CAM peut également servir au développement de tumeur expérimentale. La greffe de cellules humaines cancéreuses à la surface de la CAM est réalisée au stade embryonnaire 9 jours. Trois jours après la greffe à ED12, la formation d'une tumeur est visible à la surface de la CAM. Cette dernière peut être traitée en topique pendant les 48h qui suivent ce stade et les tumeurs sont prélevées à ED14j.

### Protocole détaillé :

Les oeufs fécondés (Ferme avicole Haas, Strasbourg, France) sont maintenus 24 h à 18°C avant de démarrer l'incubation. Ils sont ensuite incubés dans une couveuse (Maino, Italie), à 38°C avec un taux d'humidité de 45%. Les stades embryonnaires sont déterminés à partir des tables de Hamburger et Hamilton (H et H, 1951). Au stade embryonnaire 2,5 j (ED 2,5) correspondant au stade 17 de H et H, 4 mL d'albumine sont retirés à l'aide d'une seringue de 10 mL puis le contenu de chaque oeuf est extrait de sa coquille et est placé dans des nacelles de pesée hexagonales (VWR, Strasbourg, France). Ces dernières sont alors placées dans des boîtes de Pétri (VWR, Strasbourg, France) contenant de l'eau afin d'assurer le maintien d'une hygrométrie convenable et de limiter les risques de contaminations, puis les boîtes sont remises à incuber. A ce stade de développement, la membrane chorio-allantoïde commence à être vascularisée. Au stade ED9 (stade 35 de H et H), un anneau de silicone d'environ 1 cm de diamètre et d'1 mm d'épaisseur, est placé de façon à encadrer plusieurs vaisseaux sanguins. Deux approches expérimentales sont réalisées.

### 1) Traitements sur la CAM

Différents traitements (oligopeptide selon l'invention, eau distillé stérile, VEGF165, Bevacizumab) sont alors appliqués sur la membrane à l'aide d'éponges résorbables à base de gélatine bovine qui sont ensuite déposées sur la CAM (Bloxang, Laboratoire Chauvin, France). Les éponges sont découpées stérilement en cubes de 1mm d'arêtes et sont imprégnées avec le oligopeptide selon l'invention à une concentration de 250 µg/mL. Le volume d'imprégnation réalisé est de 50 µL par éponge. Un contrôle est effectué en imprégnant les éponges avec le même volume d'eau distillée stérile. Deux témoins supplémentaires de l'angiogenèse sont déposés sur la CAM, une éponge imprégnée avec le VEGF165 à 10 ng/mL (témoin positif) et une autre imprégnée avec le Bevacizumab à 250 µg/mL (témoin négatif).

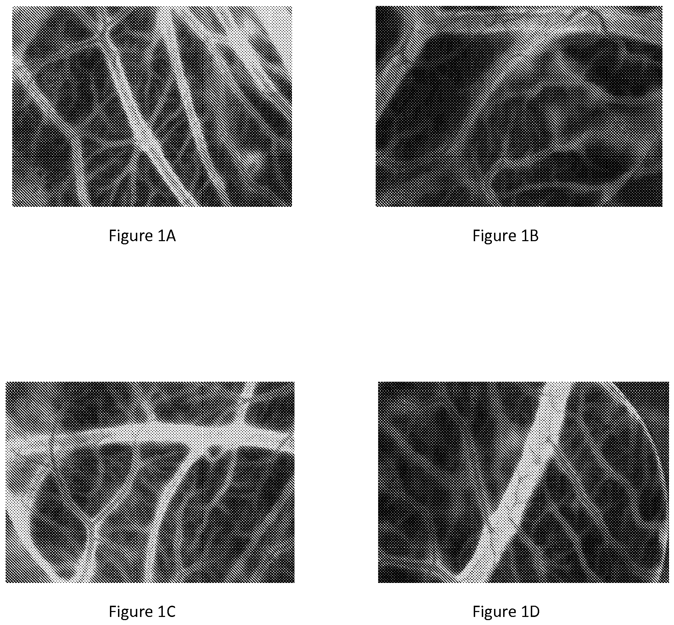

Les résultats sont présentés sur les figures 1A à 1D.

### 2) Traitements sur les tumeurs issues de greffe d'U87-MG

Pour obtenir une tumeur, les cellules U87-MG sont ensemencées à raison de 200 000 cellules dans des flasques de 75 cm2 en présence de milieu basal. Un volume contenant 4.10⁶ cellules est alors prélevé et après centrifugation 5min à 311g, le surnageant est éliminé et les cellules sont resuspendues dans 20µL de milieu différenciant qui sont déposés au centre d'un anneau en silicone préalablement appliqué sur la CAM d'un embryon à ED9. Une fois la greffe effectuée, l'embryon est remis à incuber. A ED11, les tumeurs peuvent être observées sous une binoculaire MZFL3 (Leica, Paris, France). A ce stade, les tumeurs sont soit traitées en topique (partie apicale de la tumeur) par 30µL d'olgopeptide selon l'invention à 250µg/mL, ou bien par 30µL d'eau distillée stérile jusqu'au stade ED13. Les tumeurs sont alors prélevées et des cryo-sections sont réalisées pour permettre des analyses immunohistochimiques. Le réseau angiogénique est visualisé en injectant une solution de Fluoresceine aqueuse à 1% (CHU, Limoges) dans l'artère vitelline à l'aide d'une micropipette. Les observations macroscopiques des tumeurs ainsi que la diffusion de la fluorescéine dans les vaisseaux sont effectuées avec une binoculaire à fluorescence (MZFL3, Leica, France).

Les résultats obtenus présentés sur la figure 1D montrent une inhibition de la vascularisation, après 9 heures de traitement avec l'oligopeptide selon l'invention à 250µg/mL.

L'expérience est réalisée avec injection de fluorescéine isothiocyanate (FITC) ce qui a permis de faciliter l'observation de la vascularisation de la CAM sur l'angiographie, et notamment des microvaisseaux plus profonds.

On constate que lors du traitement par le VEGF (figure 1C), un agent pro-angiogénique, la microvascularisation est dense et comprend de nombreuses ramifications vasculaires. Par opposition, l'effet anti-angiogénique de l'oligopeptide selon l'invention (figure 1D) (est comparable à celle du Bevacizumab de l'Avastin® (figure 1B) sur la microvsacularisation. Son action semble particulièrement marquée sur les microvaisseaux constituant l'arborisation vasculaire tandis que les vaisseaux les plus importants ne semblent pas altérés.

### C. Etude des effets du peptide selon l'invention sur la vascularisation tumorale

Pour obtenir une tumeur, les cellules U87-MG sont ensemencées à raison de 200 000 cellules dans des flasques de 75 cm² en présence de milieu basal.

Un volume contenant 4.10⁶ cellules est alors prélevé et après centrifugation 5min à 300g, le surnageant est éliminé et les cellules sont resuspendues dans 20µL de milieu différenciant qui sont déposés au centre d'un anneau en silicone préalablement appliqué sur la CAM d'un embryon à ED9. Une fois la greffe effectuée, l'embryon est remis à incuber.

A ED11, les tumeurs peuvent être observées sous une binoculaire MZFL3 (Leica, Paris, France) et un suivi de l'évolution de la croissance tumorale est possible jusqu'à ED14.

Les résultats sont présentés en suivant :

### a. Observation macroscopique de l'effet de l'oligopeptide selon l'invention sur l'angiogenèse tumorale dans un modèle CAM (figures 3A à 3C)

Les tumeurs développées sur la CAM sont observées sur la membrane chorio-aIlantoïdienne à l'aide d'une binoculaire de type MZFL3 (Leica, France) avant d'être prélevée.

Trois jours après la greffe des cellules tumorales, la tumeur commence à se développer et la vascularisation débute. La couleur rosée due à la présence de capillaires à la surface de la tumeur disparaît dans les 24 heures suivant le début du traitement par le peptide selon l'invention sur des tumeurs de taille réduite (1 à 2mm de diamètre) et entre 24 et 48 heures pour les tumeurs de diamètre supérieur à 2mm. Une couleur blanche nacrée apparaît à la surface de la tumeur suggérant la mise en place de processus nécrotique susceptibles de conduire à la régression tumorale (figure 3A c). Le prélèvement de la tumeur et de la CAM présente dans l'anneau (zone de traitement par le peptide selon l'invention ou le contrôle) permet de visualiser les vaisseaux qui pénètrent dans la tumeur.

Une diminution importante de la densité des vaisseaux irriguant la tumeur est constatée suite au traitement avec l'oligopeptide selon l'invention, confirmant ainsi son activité anti-angiogénique suggérée par les résultats précédents (figure 3A l).

Les valeurs correspondants aux résultats exprimés à la figure 3C sont présentées dans le tableau 2 ci-après :

**Tableau 2 : ce tableau présente les valeurs de la quantification du marquage du Ki-67 obtenues pour chaque condition de traitement (TSP-1, oligopeptide selon l'invention NX et contrôle sans traitement Ctrl) en fonction de la région tumorale étudiée (Bord et centre).**

| | | Bord1 | Bord 2 | Centre1 | Centre2 |
|---|---|---|---|---|---|
| CAM 1TSP1 | | 227 | 314 | 300 | 292 |
| CAM 2TSP1 | | 286 | 228 | 233 | 350 |
| CAM NX | | 165 | 172 | 355,5 | 383,5 |
| CAM NX | | 184 | 153 | 369 | 370 |
| CAM Ctrl | | 310 | 342 | 331 | 346 |
| CAM Ctrl | | 319 | 333 | 330 | 347 |

### b. Etude de la vascularisation et de l'expression de marqueurs tumoraux par immunohistochimie sur la tumeur traitée avec le peptide selon l'invention (figure 5)

L'endothélium vasculaire des vaisseaux de poulet est marqué à l'aide d'une lectine, la SNA-1 (Sambuccus Nigra Agglutinin-1, Vector Laboratories), couplée FITC (Fluorescein Isothiocyanate).

Après 10 min de fixation avec 200µL de PBS 1X-PFA 4% à température ambiante, les coupes sont incubées avec une solution de TBS 1X-Tween 0,05%-Trypsine 0,1% pH 7,6 pendant 10 min. Elles sont ensuite lavées 3 fois dans du PBS 1X et 100µL d'une dilution au 1/200éme de la lectine SNA-1 dans une solution de TBS 1X-Tween 0,05% à pH 7,6 est appliquée pendant 1 h. Après lavages (dont le dernier en eau distillée), les lames sont ensuite montées comme décrit dans le paragraphe précédent et observées au microscope à fluorescence. Des comptages des vaisseaux ont été effectués à partir de photos d'un grossissement x100 et dans une zone marginale s'étalant de 600 µm depuis la bordure de la tumeur. Un test statistique de type ANOVA a ensuite été réalisé pour valider la pertinence de l'effet observé. La lectine SNA-1 marque spécifiquement les cellules endothéliales de poulet constituant la paroi vasculaire. Le marquage SNA-1 sur des coupes de tumeurs non traitées montre une structure richement vascularisée avec de gros vaisseaux situés à la base de la tumeur. Après traitement par le peptide selon l'invention, le marquage SNA-1 disparaît plus spécifiquement dans la zone proche de la surface de la tumeur suggérant une disparition de la microvascularisation dans cette région.

Le co-marquage vimentine/SNA-1 a permis de compléter les observations précédentes en apportant la preuve que l'inhibition de l'angiogenèse tumorale induite par le peptide selon l'invention contribue non seulement à la régression de la microvascularisation tumorale mais également à la diminution du nombre de cellules tumorales différenciées exprimant la vimentine. Le nombre de cellules exprimant la vimentine est plus important dans les tumeurs contrôles que dans les tumeurs (figure 5).

### c. Etude de la prolifération cellulaire après traitement des tumeurs sur le modèle CAM par l'oligopeptide selon l'invention (figures 7A à 7E)

Les tumeurs sont fixées dans du PBS 1X-PFA 4% (24 h) puis déshydratées dans l'éthanol absolu puis dans du toluène pur et ensuite inclues et conservées dans un bloc de paraffine. Des coupes de 4 µm sont réalisées à partir d'un microtome et déposées sur des lames Superfrost plus, puis séchées dans une étuve à 50°C une nuit.

Pour réaliser l'immunomarquage, les coupes sont déparaffinées puis réhydratées par des bains de concentrations décroissantes d'éthanol puis rincer dans le PBS 1X.

Afin de démasquer les sites antigéniques, les lames sont incubées dans un tampon citrate à pH 7 (200 µM acide citrique ; 9,8 mM citrate de sodium) et subissent un cycle de chauffage (4x5 min à 750 W au micro-onde). Les peroxydases endogènes sont inhibées, en effectuant un bain de 10 min dans du méthanol-5 % H2O2. Après 3 lavages, la saturation en PBS-BSA 3 % puis l'incubation avec l'anticorps anti-Ki67 sont réalisées. Après rinçage au PBS 1X, un anticorps secondaire conjugué HRP est appliqué 1h à température ambiante. La révélation est effectuée avec la DAB (3,3-Diaminobenzidine). Une contre-coloration est réalisée en hématoxyline et les coupes sont montées dans une solution aqueuse avant d'être observées au microscope Leica DMRX.

Le marquage du Ki-67 est réparti de façon homogène sur l'ensemble de la tumeur contrôle (figure 7A D) tandis que dans la tumeur traitée par le peptide selon l'invention (figure 7A F) la zone périphérique n'exprime pas le marqueur Ki67. Cette région présentant un faible marquage Ki67 correspond à la bande caractérisée précédemment dans laquelle on constate une très faible expression de la vimentine et une réduction significative du nombre de vaisseaux. Ce résultat suggère donc un effet potentiel du peptide selon l'invention sur la prolifération cellulaire tumorale qui pourrait être dû à la diminution significative de la vascularisation dans la région correspondante.

L'action du peptide selon l'invention sur l'inhibition de l'angiogenèse tumorale s'accompagne donc d'une diminution de l'expression d'un marqueur des cellules tumorales (Vimentine) mis en évidence à l'aide de tumeurs expérimentales obtenues sur la CAM ainsi que d'une diminution significative de la prolifération des cellules tumorales.

### d. Analyse protéique et transcriptomique menée sur des tumeurs obtenues sur un modèle CAM et analyse des variations de sécrétion de facteurs solubles intervenant dans l'angiogenèse à la suite du traitement par l'oligopeptide selon l'invention

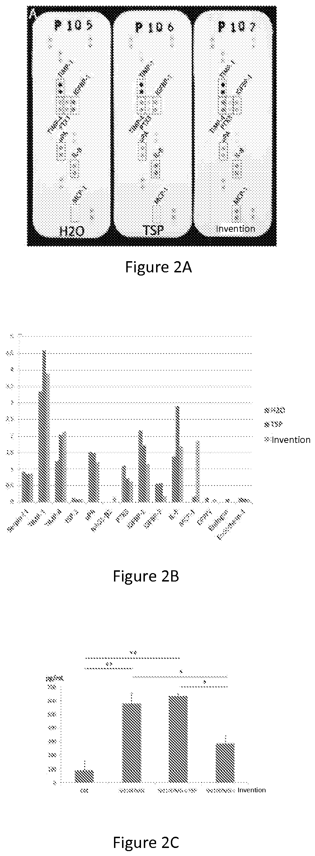

Les profils de sécrétion des cellules montrent une augmentation importante d'IL-8 dans le milieu des cellules traitées par la TSP-1, alors que l'oligopeptide selon l'invention induit une forte augmentation de MCP-1 (figures 2A et 2B) et une diminution de VEGF-A dans le surnageant des U87-MG (figure 2C). Le peptide a donc bien un effet anti-angiogénique dans un contexte associant cellules endothéliales et cellules tumorales.

Les valeurs correspondants aux résultats exprimés dans les figures 2B et 2C sont présentés dans les tableaux 3 et 4 ci-après :

**Tableau 3 : Valeurs relatives aux résultats de l'analyse proteome array des surnageants des U87-MG après traitement par H₂O (H20), TSP-1 (TSP) ou oligopeptide selon l'invention (NX) présentée dans la figure B**

| | H2O | TSP | NX |
|---|---|---|---|
| Serpin E1/PAI-1 | 0,92493716 | 0,85495271 | 0,86316564 |
| TIMP-1 | 3,34398164 | 4,59322792 | 3,88752813 |
| TIMP-4 | 1,24938431 | 2,0409603 | 2.15020158 |
| TSP-1 | 0,12252001 | 0,08481339 | 0,07983378 |
| uPA | 1,52097324 | 1,48877709 | 1,20853125 |
| VEGF | 0,77683673 | 1,39678618 | 1,02082894 |
| NRG1-p1 / HRG1-β1 | 0 | 0 | 0.14646286 |
| Pentraxin 3 (PTX3) / TSG-14 | 1,09932408 | 0,71177425 | 0,61790135 |
| IGFBP-1 | 2,17561046 | 1,7235348 | 1,1652976 |
| IGFBP-3 | 0,56194267 | 0,57127158 | 0,18556444 |
| IL-8 / CXCL8 | 1,38054145 | 2,90071833 | 1,69798791 |
| MCP-1 / CCL2 | 0 | 0,15337442 | 1,86082798 |
| DPPIV / CD26 | 0,1392225 | 0 | 0,08221834 |
| Endoglin / CD105 | 0 | 0,09235922 | 0 |
| Endothelin-1 (ET-1) | 0,12820021 | 0,09940002 | 0,09417925 |

**Tableau 4 : Valeurs relatif à l'histogramme montrant le dosage du VEGF dans le surnageant des U87-MG après traitement par H₂O (H20), TSP-1 (TSP) ou oligopeptide selon l'invention (NX) par ELISA présenté dans la figure C.**

| | May | Val1 | Val2 | pg/mL1 | pg/mL | écart type pg/ml | err std | pg/ml moyen |
|---|---|---|---|---|---|---|---|---|
| CM | 0,181 | 0,22 | 0,142 | 154,5 | 24,5 | 91,92388155 | 65,1942422 | 89,5 |
| SN U87 | 0,4745 | 0,521 | 0,428 | 656,1666667 | 501,1666667 | 109,6015511 | 77,7315965 | 578,666667 |
| SN U87 + TSP | 0,509 | 0,5 | 0,518 | 621,1666667 | 651,1666667 | 21,21320344 | 15,0448251 | 636,166667 |
| SN U87 + NX | 0,2995 | 0,333 | 0,266 | 342,8333333 | 231,1666667 | 78, 96025723 | 56,0001824 | 287 |

Concernant l'action inhibitrice directe sur les vaisseaux de l'oligopeptide selon l'invention il a été montré que la TSP-1 engendre les mêmes effets mais à un degré moindre sur ce modèle de tumeurs expérimentales issues de la CAM (figures 2A, 2B et 2C). L'oligopeptide selon l'invention est donc plus efficace que la TSP-1 dans l'inhibition de la vascularisation tumorale.

L'étude de l'expression des gènes dans des tumeurs expérimentales issues de la CAM a révélé globalement une augmentation d'un facteur 4 par rapport au contrôle de tous les gènes codant pour les récepteurs (ITGB1, PDGFRβ, KDR, TIE-1, TIE-2, CD36) étudiés dans les tumeurs traitées par l'oligopeptide selon l'invention. Ces récepteurs appartiennent à l'espèce du poulet et sont donc retrouvés sur les vaisseaux hôtes. Cette augmentation d'expression des gènes se retrouve également sur les tumeurs ayant reçu de la TSP-1 mais à moindre échelle (figure 4). Par ailleurs, la surexpression d'ITGB1 en même temps que celle de CD36 différencie le peptide selon l'invention (NX) de la TSP-1 (TSP). De plus, les niveaux d'expression observés de PDGFRB, TIE1, TEK et KDR dans les vaisseaux des tumeurs expérimentales montrent néanmoins que l'oligopeptide selon l'invention a un impact important au niveau du micro-environnement tumoral (figure 4).

Les valeurs correspondants aux résultats exprimés à la figure 4 sont présentés dans le tableau 5 ci-après :

**Tableau 5 : Le tableau représente le RQ moyen obtenu pour chaque gène selon les différentes conditions de traitement. Le RQ moyen est obtenu à partir d'un triplicat. Traitemens « con », pour contrôle, « TSP » le traitement TSP-1 avec la thrombospondine 1 et NX traitement avec le peptide selon l'invention**

| | Con | RQ TSP moy | RQ NX moy |
|---|---|---|---|
| TIMP1 | 1 | 0,44922254 | 1,23669395 |
| TIMP4 | 1 | 0,6642234 | 0,65521259 |
| ITGB1 | 1 | 1,31677272 | 4,06957648 |
| PDGFB | 1 | 0,63265723 | 2,10425551 |
| PDGFRB | 1 | 2,5435188 | 4,36553671 |
| VEGFA | 1 | 0,53918053 | 1,29771382 |
| KDR | 1 | 1,55729461 | 4,20663176 |
| ANGPT1 | 1 | 0,51056649 | 0,82414821 |
| ANGPT2 | 1 | 0,2678366 | 0,67868709 |
| TIE-1 | 1 | 1,79031205 | 3,96121105 |
| TEK | 1 | 1,38530537 | 4,53929902 |
| CD36 | 1 | 2,96481544 | 3,83116437 |
| FGF2 | 1 | 0,3429306 | 0,68597025 |
| PLAU | 1 | 1,49099798 | 1,15119235 |

### D. Etude de l'effet anti-angiogénique in vivo du peptide selon l'invention

L'expérimentation sur la souris confirme les résultats obtenus avec le modèle CAM. Dans ce cas, les tumeurs étaient issues de greffes hétérotopiques sous-cutanées au niveau de la cuisse de souris nude (figures 7B, 7C et 7E).

Les valeurs correspondants aux résultats exprimés aux figures 7B, C et E sont présentés dans les tableaux 6, 7 et 8 ci-après :

**Tableau 6**

| | Bord1 | Bord2 | Centre1 | Centre2 |
|---|---|---|---|---|
| con | 451 | 187 | 440 | 322 |
| con | 288 | 368 | 396 | 397 |
| con | 218 | 402 | 322 | 364 |
| con | 37 | 31 | 46 | 51 |
| NX | 49 | 50 | 227 | 240 |
| NX | 52 | 44 | 280 | 290 |
| NX | 27 | 4 | 123 | 238 |
| NX | 75 | 153 | 264 | 89 |
| NX | 21 | 22 | 26 | 40 |
| TSP | 302 | 246 | 297 | 282 |
| TSP | 362 | 370 | 322 | 182 |
| TSP | 53 | 42 | 66 | 53 |
| | | | | |

| | Bord Moy | Centre Moy | | |
|---|---|---|---|---|
| Ctrl | 247,75 | 292,25 | | |
| TSP | 229,166667 | 200,333333 | | |
| NX | 49,75 | 181,7 | | |

**Tableau 7**

| | bord | bord | bord | bord | centre | centre | centre | centre |
|---|---|---|---|---|---|---|---|---|
| CTRL | 37 | 31 | 33 | 46 | 46 | 51 | 42 | 55 |
| TSP1 | 53 | 42 | 39 | 45 | 66 | 53 | 63 | 56 |
| NX | 21 | 22 | 26 | 23 | 26 | 40 | 28 | 38 |

**Tableau 8**

| Volume (mm³) | HydroGel | TSP | NX |
|---|---|---|---|
| J0 | 0 | 0 | 0 |
| J5 | 43,5866889 | 33,5535167 | 46,4597889 |
| J12 | 43,5633133 | 29,9250722 | 39,0419645 |
| J17 | 73,3539325 | 49,8769811 | 70,5983644 |
| J19 | 101,617334 | 81,0688689 | 78,82028 |
| J21 | 118,098889 | 112,518586 | 135,630556 |
| J23 | 197,82 | 101,875556 | 68,0333333 |

*Les tableaux 6, 7 et 8: Ces tableaux correspondent aux valeurs brutes obtenues lors des différentes analyses menées sur les tumeurs traitées in vivo. Analyse de la prolifération (quantification marquage Ki67 (F), Analyse de la vascularisation (quantification marquage CD31) (G) et analyse de la croissance tumorale en fonction du temps (mesure du volume tumoral) (H). Traitements « con », « CTRL » et « Hydrogel » sont les conditions contrôles, « TSP » et « TSP1 » sont les traitements avec la thrombospondine 1 (TSP-1) et NX le traitement avec le peptide selon l'invention*

Les tumeurs traitées par l'oligopeptide selon l'invention diminuent de volume 48 heures après le traitement et semblent moins vascularisées que les tumeurs contrôles et celles traitées par la TSP-1 (figure 7D R). Les études histologiques et immuno-histochimiques ont montré une diminution du marquage CD31 sur la périphérie, par comparaison avec le contrôle (figure 7A L, et Figure 7C). Cette diminution de la quantité de vaisseaux s'accompagne d'une diminution de la prolifération cellulaire, visualisée par une baisse du marquage Ki-67 (figure 7B).

L'inhibition induite sur les mécanismes de vascularisation par l'oligopeptide selon l'invention, conduit à la formation d'une zone nécrotique et à la diminution de l'expression de marqueurs des cellules tumorales indifférenciées dans les tumeurs traitées par rapport aux tumeurs contrôles.

L'oligopeptide selon l'invention peut donc être utilisé en particulier pour le traitement de maladies associées à l'angiogenèse notamment du glioblastome, en particulier dans le cadre d'un ciblage multiple.

## Revendications

1. Oligopeptide **caractérisé par** la séquence suivante :
W-S-X₁-W-S-X₂-C-S-X₃-X₄-C-G (SEQ ID N°1)
dans lequel X₁, X₂, X₃ et X₄ représentent des séquences d'acides aminés consistant en de 1 à 5 acides aminés,
pour une utilisation chez l'homme ou l'animal comme médicament inhibiteur de l'angiogenèse dans le traitement d'au moins une maladie associée à l'angiogenèse choisie parmi les cancers, l'arthrite, la polyarthrite rhumatoïde, des plaques d'athérosclérose, la néovascularisation d'une greffe de cornée, des cicatrices hypertrophiques ou chéloïdes, la rétinopathie proliférante, la rétinopathie diabétique, la dégénérescence maculaire chez l'adulte, la granulation, le glaucome néovasculaire et l'uvéite.

2. Oligopeptide pour son utilisation selon l'une des précédentes revendications, **caractérisé en ce que** X₁, X₂, X₃ et X₄ représentent chacun un acide aminé, ledit acide aminé étant naturel ou non naturel.

3. Oligopeptide pour son utilisation selon l'une des précédentes revendications, **caractérisé en ce que** :
- X₁ est choisi parmi G, V, S, P et A, et/ou
- X₂ est choisi parmi G, V, S, P et A, et/ou
- X₃ est choisi parmi R, A et V, et/ou
- X₄ est choisi parmi S et P.

4. Oligopeptide pour son utilisation selon l'une des précédentes revendications, modifié par amidation, acylation, pegylation.

5. Oligopeptide pour son utilisation selon l'une des précédentes revendications, **caractérisé en ce qu'**il présente la séquence suivante :
W-S-G-W-S-S-C-S-R-S-C-G (SEQ ID N°5)

6. Oligopeptide pour son utilisation selon l'une des précédentes revendications, comme médicament inhibiteur de la néo-angiogenèse de tumeurs chez l'homme ou l'animal.

7. Oligopeptide pour son utilisation selon l'une des précédentes revendications, dans le traitement des tumeurs solides choisies parmi les tumeurs du sein, du poumon, des reins, de l'intestin, du colon, des ovaires, de la prostate

8. Oligopeptide pour son utilisation selon l'une des revendications 1 à 6, dans le traitement des tumeurs cérébrales chez l'homme ou l'animal.

9. Oligopeptide pour son utilisation selon l'une des revendications 1 à 6, dans le traitement de glioblastomes.

10. Oligopeptide pour son utilisation selon l'une des précédentes revendications, en association avec au moins un autre principe actif anti-tumoral.

11. Oligopeptide pour son utilisation selon l'une des précédentes revendications, en association avec un principe actif chimiothérapeutique anti-cancéreux.

12. Oligopeptide pour son utilisation selon l'une des précédentes revendications, en association avec un traitement de radiothérapie anti-cancéreuse.

13. Oligopeptide pour son utilisation selon l'une des précédentes revendications, **caractérisé en ce qu'**il est formulé au sein d'une composition pharmaceutique.

## Patentansprüche

1. Oligopeptid, **gekennzeichnet durch** die folgende Sequenz:
W-S-X₁-W-S-X₂-C-S-X₃-X₄-C-G (SEQ ID N°1) in der X₁, X₂, X₃ und X₄ Aminosäurensequenzen darstellen, die aus 1 bis 5 Aminosäuren bestehen,
zu deren Verwendung beim Menschen oder Tier als ein die Angiogenese hemmendes Medikament bei der Behandlung wenigstens einer Krankheit, die mit der Angiogenese zusammenhängt und aus Krebs, Arthritis, rheumatischer Polyarthritis, atherosklerotischen Plaques, der Neovaskularisation einer Hornhauttransplantation, hypertrophen oder keloiden Narben, der wuchernden Retinopathie, der diabetischen Retinopathie, der Makuladegeneration beim Erwachsenen, der Granulation, dem neovaskulären Glaukom oder der Uveitis ausgewählt ist.

2. Oligopeptid zu dessen Verwendung nach einem der vorhergehenden Ansprüche, **dadurch gekennzeichnet, dass** X₁, X₂, X₃ und X₄ jeweils eine Aminosäure darstellen, wobei die Aminosäure natürlich oder nicht-natürlich ist.

3. Oligopeptid zu dessen Verwendung nach einem der vorhergehenden Ansprüche, **dadurch gekennzeichnet, dass**:
- X₁ aus G, V, S, P und A ausgewählt ist und/oder
- X₂ aus G, V, S, P und A ausgewählt ist und/oder
- X₃ aus R, A und V ausgewählt ist und/oder
- X₄ aus S und P ausgewählt ist.

4. Oligopeptid zu dessen Verwendung nach einem der vorhergehenden Ansprüche, das durch Aminierung, Acylierung und Pegylierung modifiziert ist.

5. Oligopeptid zu dessen Verwendung nach einem der vorhergehenden Ansprüche, **dadurch gekennzeichnet, dass** dieses die folgende Sequenz darstellt:
W-S-G-W-S-S-C-S-R-S-C-G (SEQ ID N°5)

6. Oligopeptid zu dessen Verwendung nach einem der vorhergehenden Ansprüche, als hemmendes Medikament für die Neoangiogenese von Tumoren beim Menschen oder Tier.

7. Oligopeptid zu dessen Verwendung nach einem der vorhergehenden Ansprüche, bei der Behandlung von soliden Tumoren, die aus den Tumoren der Brust, der Lunge, der Niere, des Darms, des Dickdarms, der Eierstöcke oder der Prostata ausgewählt sind.

8. Oligopeptid zu dessen Verwendung nach einem der Ansprüche 1 bis 6, bei der Behandlung von Gehirntumoren beim Menschen oder Tier.

9. Oligopeptid zu dessen Verwendung nach einem der Ansprüche 1 bis 6, bei der Behandlung von Glioblastomen.

10. Oligopeptid zu dessen Verwendung nach einem der vorhergehenden Ansprüche, in Verbindung mit wenigstens einem weiteren antitumoralen Wirkstoff.

11. Oligopeptid zu dessen Verwendung nach einem der vorhergehenden Ansprüche, in Verbindung mit einem chemotherapeutischen Antikrebswirkstoff.

12. Oligopeptid zu dessen Verwendung nach einem der vorhergehenden Ansprüche, in Verbindung mit einer Antikrebsstrahlenbehandlung.

13. Oligopeptid zu dessen Verwendung nach einem der vorhergehenden Ansprüche, **dadurch gekennzeichnet, dass** dieses in einer pharmazeutischen Zusammensetzung formuliert ist.

## Claims

1. An oligopeptide **characterised by** the following sequence:
W-S-X₁-W-S-X₂-C-S-X₃-X₄-C-G (SEQ ID NO:1)
wherein X₁, X₂, X₃ and X₄ represent sequences of amino acids comprising 1 to 5 amino acids,
for use in humans or animals as an anti-angiogenic drug in the treatment of at least one illness associated with angiogenesis chosen from cancers, arthritis, rheumatoid polyarthritis, atherosclerotic plaques, neovascularisation of a corneal graft, hypertrophic scars or keloids, proliferative retinopathy, diabetic retinopathy, macular degeneration in adults, granulation, neovascular glaucoma and uveitis.

2. An oligopeptide for use according to the preceding claim, **characterised in that** X₁, X₂, X₃ and X₄ each represent an amino acid, said amino acid being natural or non-natural.

3. An oligopeptide for use according to either of the preceding claims, **characterised in that**:
- X₁ is chosen from G, V, S, P and A, and/or
- X₂ is chosen from G, V, S, P and A, and/or
- X₃ is chosen from R, A and V, and/or
- X₄ is chosen from S and P.

4. An oligopeptide for use according to any of the preceding claims, modified by amidation, acylation or pegylation.

5. An oligopeptide for use according to any of the preceding claims, **characterised in that** it has the following sequence:
W-S-G-W-S-S-C-S-R-S-C-G (SEQ ID NO:5)

6. An oligopeptide for use according to any of the preceding claims, as a drug inhibiting the neoangiogenesis of tumours in humans or animals.

7. An oligopeptide for use according to any of the preceding claims, in the treatment of solid tumours chosen from tumours of the breast, lung, kidneys, intestine, colon, ovaries or prostate.

8. An oligopeptide for use according to any of claims 1 to 6, in the treatment of brain tumours in humans or animals.

9. An oligopeptide for use according any of claims 1 to 6, in the treatment of glioblastomas.

10. An oligopeptide for use according to any of the preceding claims, in association with at least one other antitumour active principle.

11. An oligopeptide for use according to any of the preceding claims, in association with an anticancer chemotherapy active principle.

12. An oligopeptide for use according to any of the preceding claims, in association with an anticancer radiotherapy treatment.

13. An oligopeptide for use according to any of the preceding claims, **characterised in that** it is formulated in a pharmaceutical composition.


### SEQUENCE LISTING

<110> UNIVERSITE DE LIMOGES
   NEURONAX
<120> OLIGOPEPTIDES PARTICULIERS COMME MEDICAMENTS ANTI-ANGIOGENIQUES
<130> UNIV.LIM.07
<160> 7
<170> BiSSAP 1.3
<210> 1
   <211> 12
   <212> PRT
   <213> Artificial Sequence
<220>
   <223> Peptide derived from SCO-spondine
<220>
   <221> MOD_RES
   <222> 3
   <223> Amino acid sequence comprising 1 to 5 amino acids
<220>
   <221> MOD_RES
   <222> 6
   <223> Amino acid sequence comprising 1 to 5 amino acids
<220>
   <221> MOD_RES
   <222> 9
   <223> Amino acid sequence comprising 1 to 5 amino acids
<220>
   <221> MOD_RES
   <222> 10
   <223> Amino acid sequence comprising 1 to 5 amino acids
<400> 1
   Trp Ser Xaa Trp Ser Xaa Cys Ser Xaa Xaa Cys Gly
   1               5                   10
<210> 2
   <211> 4
   <212> PRT
   <213> Artificial Sequence
<220>
   <223> peptide derived from TSR
<400> 2
   Val Thr Cys Gly
   1
<210> 3
   <211> 6
   <212> PRT
   <213> Artificial Sequence
<220>
   <223> peptide derived from TSR
<400> 3
   Cys Ser Val Thr Cys Gly
   1 5
<210> 4
   <211> 4
   <212> PRT
   <213> Artificial Sequence
<220>
   <223> peptide derived from TSR
<220>
   <221> MOD_RES
   <222> 3
   <223> Xaa means one amino acid
<400> 4
   Trp Ser Xaa Trp
   1
<210> 5
   <211> 12
   <212> PRT
   <213> Artificial Sequence
<220>
   <223> oligopeptide derived from SCO-Spondin
<400> 5
   Trp Ser Gly Trp Ser Ser Cys Ser Arg Ser Cys Gly
   1 5 10
<210> 6
   <211> 12
   <212> PRT
   <213> Artificial Sequence
<220>
   <223> oligopeptide derived from SCO-Spondin
<220>
   <221> MOD_RES
   <222> 3
   <223> Gly or Val or Ser or Pro or Ala
<220>
   <221> MOD_RES
   <222> 6
   <223> Gly or Val or Ser or Pro or Ala
<220>
   <221> MOD_RES
   <222> 9
   <223> Arg or Ala or Val
<220>
   <221> MOD_RES
   <222> 10
   <223> Ser or Pro
<400> 6
   Trp Ser Xaa Trp Ser Xaa Cys Ser Xaa Xaa Cys Gly
   1               5                   10
<210> 7
   <211> 12
   <212> PRT
   <213> Artificial Sequence
<220>
   <223> oligopeptide derived from SCO-Spondin
<220>
   <221> MOD_RES
   <222> 3
   <223> Trp Ser Ser or Trp Ser Gly or Trp Ser Pro or Trp Ser Ala or Trp Ser Arg
<220>
   <221> MOD_RES
   <222> 6
   <223> Trp Ser Ser or Trp Ser Gly or Trp Ser Pro or Trp Ser Ala or Trp Ser Arg
<220>
   <221> MOD_RES
   <222> 9
   <223> Amino acid sequence comprising 1 to 5 amino acids
<220>
   <221> MOD_RES
   <222> 10
   <223> Amino acid sequence comprising 1 to 5 amino acids
<400> 7
   Trp Ser Xaa Trp Ser Xaa Cys Ser Xaa Xaa Cys Gly
   1               5                   10